# EUROPÄISCHE PATENTANMELDUNG

(11) **EP 3 772 369 A1**
(43) Veröffentlichungstag der Anmeldung: **10.02.2021**
(21) Anmeldenummer: 20189349.2
(22) Anmeldetag: 04.08.2020
(51) Int. Cl.: B01D 53/04, G01N 33/00, G01N 27/02, F25B 49/00

(54) **VERFAHREN UND VORRICHTUNG ZUR MESSUNG DES BELADUNGSZUSTANDS BEI EINER ADSORPTION**

(30) Priorität: 08.08.2019 DE 102019121456
(71) Anmelder: Vaillant GmbH, 42859 Remscheid (DE)
(72) Erfinder: Lingk, Tobias, 42799 Leichlingen (DE); Krampe-Zadler, Christof, 44628 Herne (DE); Spahn, Hans-Josef, 40699 Erkrath (DE)
(74) Vertreter: Popp, Carsten

(57) **Zusammenfassung**

Messverfahren und Messvorrichtung zur Messung des Beladungszustands bei einer Adsorption in einem Adsorber (1) eines Gehäuses einer Wärmepumpe, der der Sicherheit gegen leckagebedingt austretendes Arbeitsfluid dient, und bei dem zunächst ein hochfrequenter Wechselstrom durch eine unbeladene, leitfähige Adsorbensschicht (1) geleitet und dabei der Wechselstrom-Widerstand (E1-E4) gemessen wird, danach die Adsorbensschicht (1) beladen wird, danach der hochfrequente Wechselstrom erneut durch die dann beladene Adsorbensschicht (1) geleitet und der Wechselstrom-Widerstand gemessen wird, durch Vergleich der gemessenen Wechselstrom-Widerstände eine Beladungskennlinie erstellt wird, die Adsorbensschicht wieder entladen wird, diese Beladungskennlinie als Maß bei erneuter Beladung als Messwert für die Beladung dient.

## Beschreibung

Die Erfindung betrifft die Messung der adsorptiven Beladung von Aktivkohle und anderen leitfähigen Adsorbenzien mit gasförmigen Kohlenwasserstoffen und anderen organischen Stoffen, wie sie zur Adsorption von R290 oder sonstigen propanhaltigen Arbeitsfluiden dienen, die in Kältekreisen zum Einsatz kommen und leckagebedingt austreten könnten, sowie einer Vielzahl von anderen industriellen Einsatzzwecken.

Aktivkohle ist seit langem bekannt als Abscheidemittel für Verunreinigungen in Gasen und Flüssigkeiten. Sie wird aus Kohle gewonnen und mittels Chemikalienbehandlung und überhitztem Wasserdampf aktiviert. Im Handel ist sie in verschiedenen Formen erhältlich, üblich sind Pulver, Pellets, Gewebe, Schüttungen, gesinterte Fest- und Formkörper und viele mehr, wobei in vielen Anwendungsfällen Trägerstrukturen verwendet werden. Die Abscheidewirkung wird erzielt durch eine sehr große innere Oberfläche sowie durch gezielt eingebrachte Imprägnierungen, die bestimmte Verunreinigungen binden.

Apparativ üblich sind durchströmte Kartuschen sowie Festbetten mit Rückhaltevorrichtungen, die Aktivkohle in den oben genannten Formen enthalten. Die Auslegung solcher Apparate erfolgt, indem man die Beladungskapazität der Aktivkohle für jeden der abzuscheidenden Stoffe bestimmt, wobei die Beladungskapazität in der Regel stark temperaturabhängig ist, und die Durchströmungsmenge bestimmt. Hierbei ergibt sich im Idealfall ein Adsorptionsgleichgewicht und eine Durchbruchsfront zwischen beladenem und noch unbeladenem Adsorbens. Das gilt vor allem für Abscheidungsvorgänge aus der Gasphase.

Leider ist ein solcher Idealfall in der Praxis selten. So kennt man die Durchströmungsmenge des betreffenden Stoffes oft nicht, vor allem dann, wenn die Aktivkohle nur zur Sicherung dient und keine regelhafte Beladung erfolgen soll, oder wenn sich die Temperatur und damit die Beladungskapazität plötzlich stark ändert, was allein schon aufgrund der freiwerdenden Adsorptionswärme geschehen kann, oder wenn verschiedene abzuscheidende Stoffe sich gegenseitig verdrängen.

Praktisch muss man also bislang die Konzentration des abzuscheidenden Stoffes am Ausgang des Absorbers messen, um zu wissen, ob oder wann der Adsorber beladen ist und man kann nicht einfach erkennen, wie weit der Ladezustand innerhalb des Adsorbers bereits fortgeschritten ist. Das ist insofern unbefriedigend, als man möglicherweise eine gewisse Vorlaufzeit für einen Filterwechsel benötigt und abschätzen möchte, wann der Filterwechsel erforderlich wird, und zwar bevor der abzuscheidende Stoff durchbricht.

Die Aufgabe der Erfindung ist daher, eine Messvorrichtung bereitzustellen, mit der der Beladungszustand und die zeitliche Beladungszustandsänderung von R290 oder anderen propanhaltigen Arbeitsfluiden einer Wärmepumpe in einer Adsorbensschüttung kontinuierlich oder diskontinuierlich festgestellt werden kann, wobei die Adsorbensschüttung mit dem Gehäuse einer Wärmepumpe strömungstechnisch verbunden ist. Hierbei wird von der Impedanz Gebrauch gemacht, wobei einige Messverfahren bekannter Stand der Technik sind.

In der Veröffentlichung "Jürgen U. Keller, Rainer Staudt; Gas Adsorption Equilibria; Springer Science + Business Media, Boston 2010, Seiten 289 ff." werden eine Messvorrichtung und ein Verfahren skizziert, mit dem man derartige Beladungszustände mittels Impedanz- und Permittivitätsmessungen zwar grundsätzlich bestimmen kann, um etwa Adsorptionsgleichgewichte und Adsorptionswärmen zu ermitteln. Die dort vorgestellten Apparaturen und Auswertungsverfahren eignen sich aber weder für dynamische Messungen noch für den industriellen Serieneinsatz, sondern nur für stationäre Messungen zum Vergleich der Eignung verschiedener Adsorbenzien für unterschiedlichste Adsorbate und Gasmischungen. Die Zielsetzung und somit die technische Ausführung sind daher völlig verschieden. Eine direkte Anwendung scheidet daher aus.

Die DE 100 18 745 A1 beschreibt ein Verfahren und eine Vorrichtung zur Messung von komplexen elektrischen Widerständen und Impedanzspektren in zeitlich schneller Abfolge zum Ziel der kontinuierlichen Charakterisierung der elektrischen Eigenschaften einer Probe, also mittels hoch-zeitauflösender Impedanzspektroskopie. Beschrieben werden zunächst das Frequenzdomänenverfahren und das Zeitdomänenverfahren, wobei das Messverfahren eine Weiterentwicklung des Zeitdomänenverfahrens nutzt. Sodann wird das Messverfahren auf Lipidmembranen angewendet. Eine Anwendung auf Schüttungen von Festkörpern und Gasen ist nicht vorgesehen. Es wird auch nicht beschrieben, wie eine hierfür erforderliche Kalibrierung für eine Langzeitmessung vorzunehmen wäre.

Die DE 10 2017 207 710 A1 beschreibt ein Kalibrierungsverfahren, seine Anwendung und Vorrichtung zur Durchführung des Verfahrens. Hierbei wird die Kalibrierung von MOX-Sensoren behandelt, wobei MOX für Metalloxid steht, und wobei MOX-Sensoren von einer Vielzahl von Parametern abhängen, die eine direkte Messung von gasförmigen Substanzen erschweren. Hierbei wird zunächst ein Impedanz-Spektrum des Gassensors in einem Gasgemisch in Abwesenheit eines Analyten bestimmt, um eine Basislinie zu ermitteln, danach wird das Impedanz-Spektrum des Gassensors in einem Gasgemisch in Anwesenheit mindestens eines Analyten bestimmt. Zum Glück wird der MOX-Sensor hierdurch nicht beschädigt und er muss auch nicht regeneriert werden. Kalibrierbar sind eine Vielzahl chemischer Stoffe, auch Alkanen, die jedoch gasförmig sein müssen. Zu bemerken ist, dass nicht die Beladung des MOX-Sensors gemessen und kalibriert wird, sondern seine Reaktion auf den Anteil eines gasförmigen Analyten in einem Gas.

In der Veröffentlichung "M.F. Smiechowsky, W.B. Feaver, AC Impedance Monitoring of Activated Carbon Filter Contamination, ECS Transactions, 41 (28) 57-67 (2012) wird die Impedanzmessung von Aktivkohlefiltern bezüglich Beladung mit NO₂ und SO₂ beschrieben, die Elektroden sind hierbei am Anfang und am Ende der Versuchsstrecke angeordnet. Es zeigte sich, dass unter bestimmten Umständen Durchbruchskurven in Abhängigkeit der Konzentration der Schadgase in einem Luftstrom erkennbar wurden, wobei die Impedanz um bis zu 30 Prozent gegenüber unbeladener Aktivkohle abfiel. Ergebnisse über den räumlichen Verlauf dieser Durchbruchskurven sind so jedoch nicht messbar, im praktischen Fall kann man daher nie wissen, wie weit man noch vom Durchbruch entfernt ist, man misst ihn erst, wenn er erfolgt, eine Vorlaufzeit erhält man nicht.

Die Erfindung löst die Aufgabe durch ein Messverfahren zur Messung des Beladungszustands bei einer Adsorption mithilfe von Impedanzmessung und ist besonders gut für Aktivkohle, ansonsten aber auch für jedes andere Adsorbens, welches als Dielektrikum wirkt, anwendbar. Als Impedanz wird allein die elektrische Impedanz ermittelt. Hierbei wird zunächst eine Kalibrierung für den abzuscheidenden Stoff oder die Stoffmischung vorgenommen und nachfolgend das Messverfahren für Vergleichsmessungen eingesetzt, das Messverfahren besteht also immer aus diesen beiden Schritten.

Die Erfindung löst die Aufgabe der Messung des Beladungszustands bei einer Adsorption von leckagebedingt ausgetretenem Alkan aus dem Arbeitsfluid einer Wärmepumpe durch ein Messverfahren, bei dem
- in einem ersten Schritt mittels einer Kalibrierung vorgesehen wird, dass
- zunächst an einer ersten Elektrode, die eine unbeladene Adsorbensschicht einer Adsorbensschüttung berührt, eine hochfrequente Wechselspannung angelegt wird, deren Frequenz während der Messung in einem weiten Bereich durchfahren wird,
- dann an einer zweiten Elektrode, die dieselbe Adsorbensschicht der Adsorbensschüttung berührt und der ersten Elektrode gegenüber angeordnet ist, die Spannung erfasst wird, und dabei der Spannungsabfall und der Stromfluss zwischen den beiden Elektroden bestimmt werden,
- danach die Adsorbensschicht schrittweise beladen wird,
- während des Beladungsvorgangs der hochfrequente Wechselstrom erneut durch die dann beladene Adsorbensschicht geleitet und der Wechselstrom-Widerstand gemessen wird,
- durch Vergleich der gemessenen Wechselstrom-Widerstände eine Beladungskennlinie erstellt wird,
- im zweiten Schritt der Messungsvorbereitung wird vorgesehen, dass die Adsorbensschicht wieder entladen wird,
- und die Beladungskennlinie in einer Auswertungseinheit als Messgröße für die Beladung dient.

Ein solches Alkan ist beispielsweise Propan als Hauptbestandteil von R290, aber auch die Arbeitsfluid R600a und R1270 sind Alkane, die einzeln oder in Mischungen im Arbeitsfluid von Wärmepumpen eingesetzt werden und die beim Austreten entzündliche Gemische mit Luft bilden können.

Im Unterschied zum Stand der Technik besteht die Adsorbensschüttung in der Regel aus mehreren Schichten, die getrennt voneinander mit paarweise zueinander angeordneten Elektroden gemessen werden. Die Beladungskennlinie kann dann als Maß bei erneuter Beladung für alle baugleichen Wärmepumpen, Adsorbenzien, Adsorptive und Adsorbate verwendet werden, in denen die gleiche Anordnung der Messtechnik und die gleiche Adsorbensschüttung zum Einsatz kommt.

Ein solcher Wechselstrom-Widerstand wird als Impedanz bezeichnet. Sofern das Adsorbens in einem durchströmten Behälter eingefüllt ist, müssen der Behälter und seine Einbauten aus elektrisch nichtleitendem Material gefertigt sein, etwa aus Keramik oder Kunststoff, damit keine Ströme durch Gehäuse und Einbauten fließen können, die das Messergebnis verfälschen. Je nach verwendeten Adsorbenzien, Adsorptiven und Adsorbaten können mehrere Wechselstromfrequenzen oder ein kontinuierlich durchlaufenes Wechselstromspektrum zur Bestimmung der Kennlinie zum Einsatz kommen. Die Frequenzabhängigkeit der Kennlinien ist dabei charakteristisch und somit spezifisch für jedes unterschiedliche Adsorbens sowie auch für Mischungen von Adsorbenzien. Dies liegt an der Permittivität der Adsorbenzien, die auch als dielektrische Leitfähigkeit bezeichnet wird. Sofern große Temperaturunterschiede zu berücksichtigen sind, ist die Temperaturabhängigkeit der dielektrischen Leitfähigkeit bei der Interpretation der Messergebnisse entsprechend zu berücksichtigen.

Sofern ein Behälter durchströmt wird, ist in einer Ausgestaltung des Verfahrens vorgesehen, dass mehrere Adsorbensschichten über die Lauflänge des Strömungsweges nacheinander mit dem hochfrequenten Wechselstrom beschickt werden. Auf diese Weise kann während des Beladungsvorgangs erkannt werden, in welcher Adsorbensschicht gerade eine Beladung stattfindet und wie hoch die Beladung jeder der Adsorbensschichten aktuell ist. Es ergibt sich ein zeitlicher und örtlicher Verlauf der Beladung, und je nachdem, auch von Verdrängungseffekten unterschiedlicher Adsorptive oder Desorptionseffekten aufgrund von Erwärmung.

Die Erfindung löst die Aufgabe auch durch eine Messvorrichtung mit Sensoren, die aus Elektroden gebildet werden, von denen je zwei an einen Wechselstromgenerator angeschlossen sind, der ein hochfrequentes elektrisches Wechselfeld erzeugt, sowie an einen Stromkreis angeschlossen sind, dessen Wechselstromwiderstand gemessen wird. Diese Elektroden werden in direkten Kontakt mit dem Adsorbens gebracht und sie sind gegenüber dem Gehäuse sowie anderen verbauten Elektroden jeweils isoliert.

In einer Ausgestaltung werden je zwei ringförmige Elektroden konzentrisch zueinander angeordnet in der Weise, dass zwischen ihnen ein Zwischenraum für Adsorbens bleibt. Die beiden ringförmigen Elektroden werden axial von der Gasströmung durchströmt und lassen dabei im Innenraum der inneren Elektrode und im Außenraum um die äußere Elektrode Raum für weiteres Adsorbens frei, insofern davon ausgegangen wird, dass sich die Gasströmung durch das Adsorbens im Zwischenraum und außerhalb davon gleich verteilt und die Beladungen identisch sind.

In einer Ausgestaltung werden je zwei Elektroden einander gegenüber angeordnet in der Weise, dass zwischen ihnen ein Zwischenraum für Adsorbens bleibt. Eine solche Anordnung ist vorzuziehen, wenn es sich bei der apparativen Filtergestaltung nicht um runde Kartuschen; sondern um Rechteckkanäle handelt.

Bei diesen Ausgestaltungen ist darauf zu achten, dass die Elektroden groß gegenüber den Partikeldurchmessern der Schüttung sind, damit Unregelmäßigkeiten an den Berührungsflächen statistisch ausgeglichen werden und nicht zu Messfehlern führen können.

In weiteren Ausgestaltungen wird vorgesehen, dass über die Lauflänge mehrere Elektroden angeordnet sind. Diese können paarweise gegenüberliegend angeordnet sein, müssen dies aber nicht. Es können auf diese Weise auch Verschaltungen erzeugt werden, bei denen der Wechselstrom nicht lotrecht zur Gasströmung, sondern schräg dazu zwischen den Elektroden durch das Adsorbens geleitet wird. Es können auch mehrere verschiedene Bauarten von Elektroden miteinander kombiniert werden.

Für jede der Adsorbensschichten in der Adsorbensschüttung ist jeweils ein Elektrodenpaar vorgesehen. Die verschiedenen Adsorbensschichten können ohne Trennmittel ineinander übergehen, sie können aus verschieden dotierten Aktivkohlen bestehen.

Die Erfindung umfasst auch einen Adsorber für ein gasförmiges, strömendes Medium, der mit einer Messvorrichtung wie oben beschrieben ausgestattet ist. Vorzugsweise ist dieser Adsorber mit einer Adsorbensschüttung aus mehreren Adsorbensschichten ausgestattet, wobei jede der Adsorbensschichten mit jeweils paarweise einander zugeordneten Elektroden ausgestattet ist. Der Adsorber kann als Rundrohr oder als Rechteckkanal ausgeführt sein, selbstverständlich sind auch andere Querschnitte möglich.

So kann bei einem runden Adsorptionsfilter je eine Anordnung von ringförmigen, konzentrischen Elektroden in der Nähe der Einströmung und der Ausströmung platziert werden. Schaltet man im Wechsel jeweils die Elektroden am Eingang mit denen am Ausgang in einen Stromkreis, ergibt sich der Wechselstromwiderstand für die Lauflänge in axialer Richtung und es ergibt sich ein Maß für die Durchschnittsbeladung, während bei Schließung der Stromkreise jeweils am Eingang und am Ausgang zwischen innerem und äußerem Ring die Beladung am Anfang und am Ende ergibt. Platziert man weitere Elektroden über die Lauflänge, kann die Beladung räumlich feiner aufgelöst werden.

Bei einem Rechteckkanal kann dasselbe Vorgehen mit flächigen Elektroden erfolgen, bei denen der Wechselstrom abwechselnd diagonal durch das Adsorbens geleitet wird.

Die Erfindung wird nachfolgend anhand von Prinzipskizzen näher erläutert. Hierbei zeigen:
Fig. 1: einen Adsorber mit einer Messvorrichtung,
Fig. 2 einen Schnitt durch eine erste Ausführungsvariante der Messelektroden,
Fig. 3 einen Schnitt durch eine zweite Ausführungsvariante der Messelektroden,
Fig. 4a eine erste Verschaltungsanordnung des Messstromkreises,
Fig. 4b eine zweite Verschaltungsanordnung des Messstromkreises.

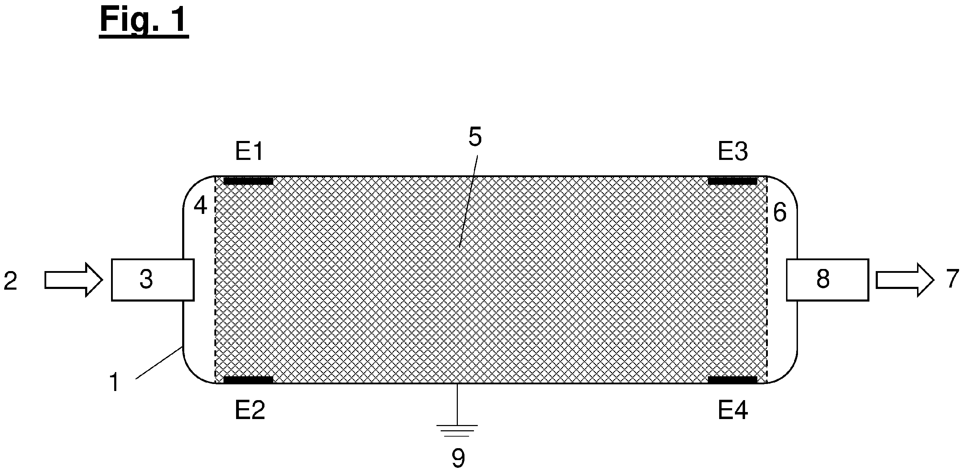

Fig. 1 zeigt einen Adsorber 1, in den ein beladenes Gas 2 durch den Gaseinlass 3 einströmt. Dort tritt das beladene Gas durch das Haltesieb 4 in das Aktivkohlebett 5 ein und strömt unter Abgabe abzuscheidender Stoffe, z.B. Propan enthaltend im Kältemittel R290, durch das Aktivkohlebett hindurch. Im Eingangsbereich des Aktivkohlebettes 5 sind die Elektroden E1 und E2 gegenüberliegend angeordnet, im Ausgangsbereich die Elektroden E3 und E4. Nach der Passage eines weiteren Haltesiebes 6 tritt das gereinigte Gas 7 aus dem Gasaustritt 8 aus. Der Adsorber ist aus nichtleitendem Material gefertigt und mit einer Erdung 9 versehen.

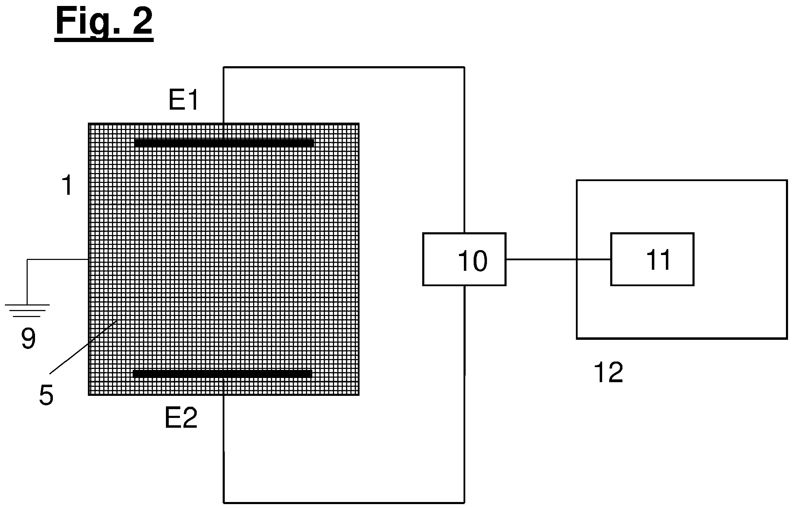

Fig. 2 zeigt in einer Schnittansicht eine Ausführungsvariante mit den konzentrischen Elektroden E1 als äußeren Ring und E2 als inneren Ring in einem Adsorber 1 mit kreisrundem Querschnitt und einem Aktivkohlebett 5, welches auch den Zwischenraum zwischen den Elektroden E1 und E2 ausfüllt. Die Elektroden E1 und E2 sind an einen Stromkreis mit dem Oszillator 10 angeschlossen der ein variables elektrisches Wechselfeld bereitstellt. An den Oszillator 10 ist ein Frequenzzähler 11 angeschlossen, der die ermittelten Daten an eine MCU (Micro Controller Unit) 12 weiterleitet.

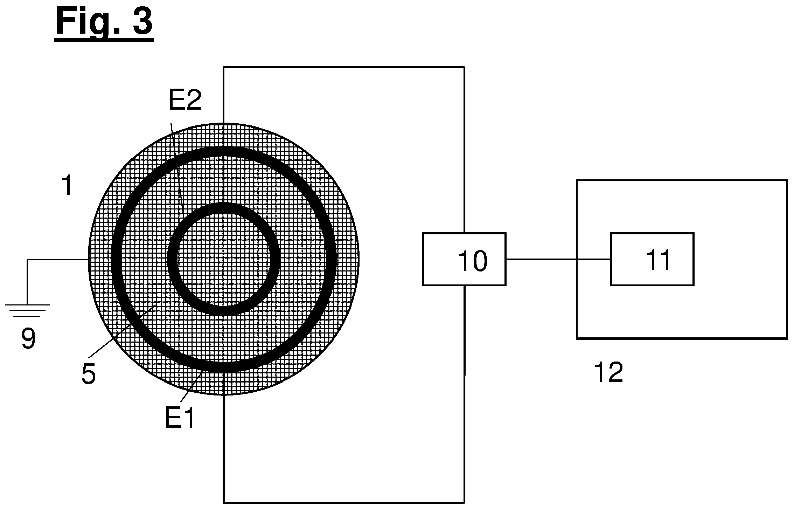

Fig. 3 zeigt in einer Schnittansicht eine Ausführungsvariante mit den plattenförmigen, einander gegenüber angeordneten Elektroden E1 und E2 in einem Adsorber 1 mit Rechteckquerschnitt und einem Aktivkohlebett 5, welches auch den Zwischenraum zwischen den Elektroden E1 und E2 ausfüllt. Die Elektroden E1 und E2 sind an einen Stromkreis mit dem Oszillator 10 angeschlossen der ein variables elektrisches Wechselfeld bereitstellt. An den Oszillator 10 ist ein Frequenzzähler 11 angeschlossen, der die ermittelten Daten an eine MCU (Micro Controller Unit) 12 weiterleitet.

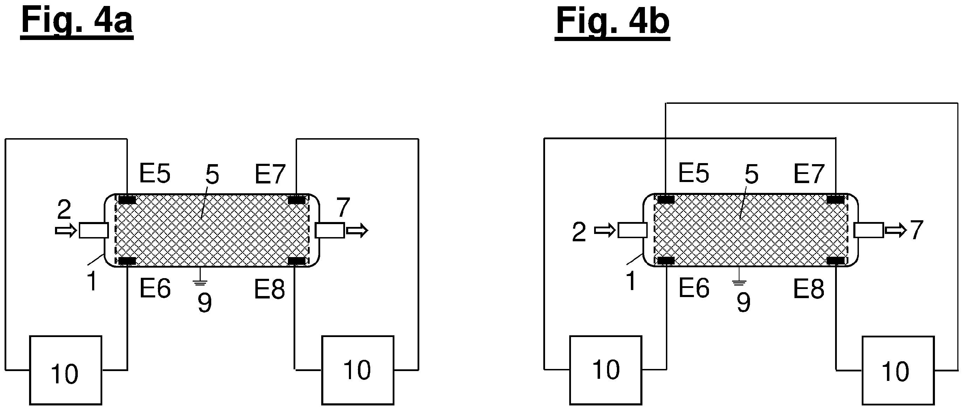

Fig. 4a und 4b zeigen zwei Verschaltungsanordnungen. Fig. 4a zeigt eine Verschaltung, bei der die Elektroden E1 und E2 einen ersten und E3 und E4 einen zweiten Stromkreis bilden. Im unbeladenen Zustand ergeben sich in beiden Fällen identische Wechselstromwiderstände. Im Verlauf einer Beladung mit Adsorbat ändert sich der Wechselstromwiderstand im Stromkreis von E1 mit E2, während der zweite Stromkreis mit E3 und E4 unverändert bleibt. Sobald die Durchbruchskurve das Ende des Aktivkohlebettes erreicht hat, gleicht sich der Wechselstromwiderstand dem ersten Wechselstromkreis an, eine entsprechende Warnung kann dann erfolgen.

Fig. 4b zeigt eine Schaltung an, bei der die Stromkreise von E1 mit E4 und E2 mit E3 geschlossen werden. Der Wechselstromwiderstand wird dann über die gesamte Lauflänge des Aktivkohlebettes bestimmt, während der Beladung steigt der Widerstand kontinuierlich an und ergibt ein Maß für den Beladungszustand, wobei im Regelfall die beiden Wechselstromkreise dieselben Werte angeben, wenn die Anordnung symmetrisch ist.

Selbstverständlich können auch weitere Elektroden angebracht werden, um die räumliche Beladung höher aufzulösen, wenn dies gewünscht ist. Da die jeweiligen Messläufe nur jeweils wenig Zeit beanspruchen, können sie nacheinander ausgeführt werden, auch die beiden gezeigten Verschaltungen können alternierend durchgeführt werden, sobald sich im Stromkreis von E1 mit E2 ein Beladungssignal zeigt.

### Bezugszeichenliste

- E1: Elektrode
- E2: Elektrode
- E3: Elektrode
- E4: Elektrode
- 1: Adsorber
- 2: beladenes Gas
- 3: Gaseinlass
- 4: Haltesieb
- 5: Aktivkohlebett
- 6: Haltesieb
- 7: gereinigtes Gas
- 8: Gasaustritt
- 9: Erdung
- 10: Oszillator
- 11: Frequenzzähler
- 12: MCU

## Patentansprüche

1. Messverfahren zur Messung des Beladungszustands bei einer Adsorption von leckagebedingt ausgetretenem Propan aus dem Arbeitsfluid einer Wärmepumpe,
**dadurch gekennzeichnet, dass**
- in einem ersten Schritt mittels einer Kalibrierung vorgesehen wird, dass
- zunächst an einer ersten Elektrode, die eine unbeladene Adsorbensschicht einer Adsorbensschüttung berührt, eine hochfrequente Wechselspannung angelegt wird, deren Frequenz während der Messung in einem weiten Bereich durchfahren wird,
- dann an einer zweiten Elektrode, die dieselbe Adsorbensschicht der Adsorbensschüttung berührt und der ersten Elektrode gegenüber angeordnet ist, die Spannung erfasst wird, und dabei der Spannungsabfall und der Stromfluss zwischen den beiden Elektroden bestimmt werden,
- danach die Adsorbensschicht (5) schrittweise beladen wird,
- während des Beladungsvorgangs der hochfrequente Wechselstrom erneut durch die dann beladene Adsorbensschicht (5) geleitet und der Wechselstrom-Widerstand gemessen wird,
- durch Vergleich der gemessenen Wechselstrom-Widerstände eine Beladungskennlinie erstellt wird,
- im zweiten Schritt der Messungsvorbereitung die Adsorbensschicht (5) wieder entladen wird,
- und die Beladungskennlinie in einer Auswertungseinheit als Messgröße für die Beladung dient.

2. Messverfahren nach Anspruch 1, **dadurch gekennzeichnet, dass** die Beladungskennlinie für alle baugleichen Apparate, Adsorbensschüttungen, Adsorbenzien, Adsorptive und Adsorbate verwendet wird.

3. Messverfahren nach einem der Ansprüche 1 oder 2, **dadurch gekennzeichnet, dass** mehrere Wechselstromfrequenzen zur Bestimmung der Beladungskennlinie benutzt werden.

4. Messverfahren nach einem der Ansprüche 1 bis 3, **dadurch gekennzeichnet, dass** ein kontinuierliches Spektrum von Wechselstromfrequenzen zur Bestimmung der Beladungskennlinie benutzt wird.

5. Messverfahren nach einem der Ansprüche 1 bis 4, **dadurch gekennzeichnet, dass** ein mit einem Adsorbat beladener Gasstrom (2) durch Schichten eines Adsorbens geleitet wird, wobei mindestens zwei Adsorbensschichten über die Lauflänge des Strömungsweges des Gasstroms mit hochfrequentem Wechselstrom nacheinander beschickt werden.

6. Messvorrichtung zur Messung des Beladungszustands bei einer Adsorption, **dadurch gekennzeichnet, dass** je zwei Elektroden (E1, E2, E3, E4) mit dem Adsorbens in Kontakt gebracht werden, die beide in einem Wechselstromkreis an einen Wechselstromgenerator (10) angeschlossen sind, wobei der Wechselstromkreis eine Messvorrichtung (11) für den Wechselstromkreis aufweist, und zwischen den beiden Elektroden (E1, E2, E3, E4) ein mit Adsorbens gefüllter Zwischenraum vorgesehen ist.

7. Messvorrichtung nach Anspruch 6, **dadurch gekennzeichnet, dass** die beiden Elektroden (E1, E2, E3, E4) als konzentrische Ringe ausgeführt sind.

8. Messvorrichtung nach Anspruch 6, **dadurch gekennzeichnet, dass** die beiden Elektroden (E1, E2, E3, E4) als gegenüberliegende Platten ausgeführt sind.

9. Messvorrichtung nach einem der Ansprüche 6 bis 8, **dadurch gekennzeichnet, dass** eine Vielzahl von Elektroden (E1, E2, E3, E4) paarweise über die Lauflänge des Strömungswegs des Gasstroms vorgesehen werden, die nacheinander und in wechselnden Verschaltungen mit Wechselstrom beschickt werden.

10. Adsorber (1) für ein gasförmiges, strömendes Medium, **dadurch gekennzeichnet, dass** er mit einer Messvorrichtung nach einem der Ansprüche 6 bis 9 ausgestattet ist.

11. Adsorber (1), nach Anspruch 10, **dadurch gekennzeichnet, dass** er mit einer Adsorbensschüttung aus mehreren Adsorbensschichten ausgestattet ist, wobei jede der Adsorbensschichten mit jeweils paarweise einander zugeordneten Elektroden ausgestattet ist.

12. Adsorber (1) nach einem der Ansprüche 10 oder 11, **dadurch gekennzeichnet, dass** er als Rundrohr ausgeführt ist und die Elektroden als konzentrische Ringe ausgeführt sind.

13. Adsorber (1) nach einem der Ansprüche 10 oder 11, **dadurch gekennzeichnet, dass** er als Rechteckrohr ausgeführt ist und die Elektroden als gegenüberliegende Platten ausgeführt sind.

14. Verwendung eines Messverfahrens gemäß einem der Ansprüche 1 bis 5 in einem Adsorber (1) nach einem der Ansprüche 10 bis 13, **dadurch gekennzeichnet, dass** das Adsorbens Aktivkohle und das Adsorptiv Propan ist.
